# FASCICULE DE BREVET EUROPEEN

(11) **EP 2 455 014 B1**
(45) Date de publication et mention de la délivrance du brevet: **12.08.2015**
(21) Numéro de dépôt: 10306265.9
(22) Date de dépôt: 17.11.2010
(51) Int. Cl.: A61B 17/17, A61B 17/74

(54) **Dispositif implantable pour le traitement préventif ou curatif de fractures du fémur, ancillaire associé**
Implantierbare Vorrichtung zur präventiven oder kurativen Behandlung von Oberschenkelknochenbrüchen, und entsprechendes chirurgisches Instrument
Implantable device for preventive or interventive treatment of femur fractures, associated ancillary device

(43) Date de publication de la demande: 23.05.2012
(73) Titulaire: Hyprevention, 33604 Pessac (FR)
(72) Inventeur: Szpalski, Marek, B-1180, BRUXELLES (BE); Gunzburg, Robert, B-2600, BERCHEM (BE); Aebi, Max, CH-2503, BIENNE (CH); Corp, Stéphane, 75005, PARIS (FR); Vienney, Cécile, 33830, BELIN BELIET (FR)
(74) Mandataire: Cenatiempo, Julie Adeline Anne

(56) Documents cités:
- EP-A1- 0 617 927
- WO-A1-01/54598
- WO-A2-2005/053545
- US-A1- 2007 225 714
- US-A1- 2010 023 011

## Description

La présente invention a pour objet un dispositif implantable pour le traitement préventif ou curatif de fractures du fémur, plus particulièrement de l'articulation coxofémorale.

L'invention couvre l'ancillaire associé.

De façon générale les os sont constitués dans la partie intérieure d'une matière spongieuse présentant un fort degré de porosité entourée d'une matière beaucoup plus rigide et résistante mécaniquement dite cortex ou os cortical. Dans le squelette, l'articulation de la hanche est une articulation soumise à des efforts intenses. Le fémur se prolonge du bassin jusqu'au genou.

Ce fémur comprend une articulation coxofémorale composée d'une tête fémorale, d'un col, des petit et gros trochanters qui relient la tête et le col du fémur au corps de l'os long, la diaphyse.

Cet os est le plus long et le plus solide du corps humains puisqu'il reprend le poids du corps.

L'extrémité proximale du fémur est particulièrement soumise aux phénomènes d'ostéoporose, ce qui diminue fortement la résistance mécanique de cet ensemble proximal.

Ceci est particulièrement fréquent chez les sujets âgés.

De ce fait, la fracture se produit particulièrement dans les deux zones du col du fémur et dans les régions trochantériennes.

On trouve les fractures dites : fracture sous capitale du col du fémur, fracture transcervicale du col du fémur, fracture pertrochantérienne, fracture soustrochantérienne ou fracture du petit trochanter.

Ces fractures sont la cause d'une majorité de décès parmi les personnes âgées, du fait des complications qu'elles engendrent.

On sait que, au-delà d'un certain âge, environ 50 ans, la masse osseuse et la densité osseuse peuvent diminuer dans certaines zones et notamment dans la zone de la tête fémorale tandis que les os longs voient leur résistance augmenter.

Parallèlement la réponse neuromusculaire diminue si bien que les muscles chargés de reprendre des efforts soudains et de dissiper le pic d'énergie cinétique généré n'apportent plus la compensation nécessaire.

De ce fait, l'effort se concentre sur l'articulation coxofémorale fragilisée, ce qui conduit aux différents types de fractures ci-dessus évoquées, en fonction du type de choc, d'effort, de l'orientation, de la concentration et de très nombreux paramètres.

Cette fragilité est engendrée par la géométrie particulière de cette tête fémorale supportée par le col du fémur, en déport par rapport à l'axe longitudinal du corps de l'os long du fémur.

Les moyens mis en oeuvre dans l'art antérieur consiste à réparer la fracture, en réduisant la fracture si nécessaire, en stabilisant et en fixant la partie cassée. Le traitement connu consiste à recourir à des systèmes composés de clous intra médullaires et de vis de compression placées dans la tête fémorale.

La chirurgie connue fait aussi appel à des systèmes combinant des plaques et des vis de pression ou même à l'injection de ciment dans les zones concernées.

De façon surprenante, on constate que l'art antérieur est très pauvre en ce qui concerne la prévention des fractures par augmentation de la résistance mécanique de la tête fémorale.

Le brevet US 2007/0225714 A1 montre un dispositif implantable pour le traitement curatif de l'articulation coxofémorale conforme au préambule de la revendication 1, mais ne permettant pas de reprise d'effort à la compression.

On peut citer un brevet US 6 679 890 qui prévoit le recours à une technique hybride utilisant un implant creux unique. Cet implant creux est introduit suivant l'axe du col du fémur puis du ciment biocompatible est injecté dans cet implant creux, ledit ciment se diffusant périphériquement audit implant.

Il est aussi prévu la possibilité de doubler l'intervention par un second implant indépendant du premier, suivant une direction longitudinale suivant l'axe de l'os long constituant le fémur.

Les deux tubes ne sont pas liés entre eux, le second tube étant utilisé comme un guidage.

La demande de brevet porte en effet sur un embout biocompatible en un matériau spécifique de façon à assurer un ancrage satisfaisant du premier implant proximal, suivant l'axe longitudinal du col.

Ce dispositif est adapté pour la réparation de fracture mais il n'est prévu aucune application dans le traitement préventif des fractures.

Si l'objet de la présente demande de brevet est applicable à la réparation de fracture de la hanche plus particulièrement des différents types de fractures de l'articulation coxofémorale, il se trouve que la présente invention vise particulièrement une application préventive de renforcement de la résistance de cette tête fémorale chez des sujets à risques.

La présente invention propose de renforcer la résistance mécanique de l'articulation coxofémorale pour prévenir les fractures ou lésions de la tête fémorale, du col du fémur ou de la région trochantérienne.

A cet effet la présente invention, définie dans la revendication 1, recourt à un ensemble de deux implants sécants et à des moyens de solidarisation des deux implants entre eux au point d'intersection.

De plus, ces deux implants sont tous deux sécants avec l'axe longitudinal du corps de l'os long.

Le point d'intersection est sensiblement dans la zone du pied du col.

Plus particulièrement, le premier implant est orienté suivant l'axe du col et le second plus incliné passant par le point d'intersection des deux implants.

Le premier implant a une fonction de raidisseur et le second implant a une fonction de jambage.

L'invention est maintenant décrite en détail suivant différents modes de réalisation, non limitatifs, en regard des dessins annexés, ces dessins illustrant les différents modes de réalisation.

On trouve les différentes figures suivantes :
- Figure 1 : une vue schématique de l'articulation coxofémorale.
- Figure 2 : une vue schématique du dispositif implantable selon la présente invention.
- Figure 3 : une vue schématique du dispositif implantable selon la présente invention, montré de façon isolée,
- Figure 4 : une vue en perspective d'un premier mode de réalisation avec une liaison par emboîtement du second implant formant jambage dans le premier implant formant raidisseur,
- Figure 5 : une vue en perspective d'un second mode de réalisation avec une liaison par emboîtement du second implant formant jambage dans le premier implant formant raidisseur,
- Figures 6A, 6B et 6C : des vues respectives en perspective complète, en coupe longitudinale, en détail de l'entretoise, d'un troisième mode de réalisation avec une liaison par encastrement fileté du second implant formant jambage dans le premier implant formant raidisseur.
- Figure 7 : une vue en perspective d'un implant, et
- Figures 8A à 8F : des vues des étapes d'un protocole d'implantation du dispositif selon la présente invention.

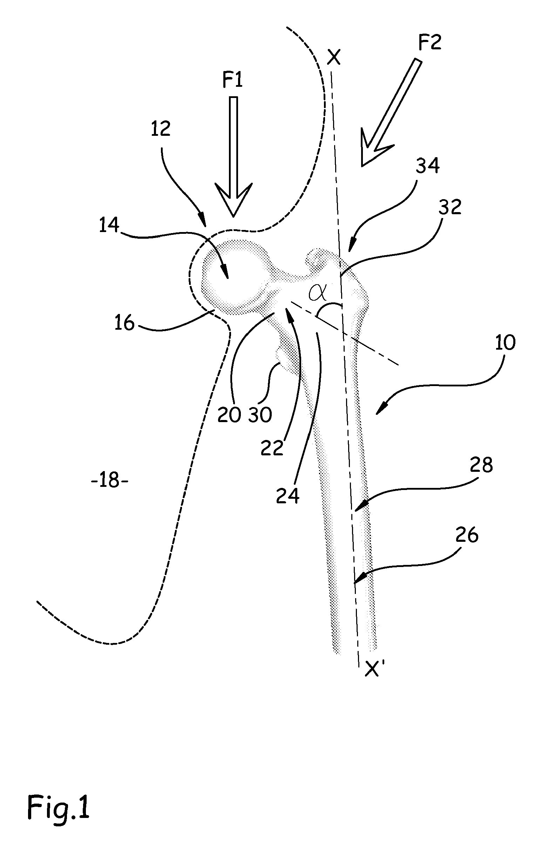

Sur la figure 1, on a représenté la partie proximale d'un fémur 10 avec son articulation 12 coxofémorale. Le fémur représenté est le fémur de la jambe gauche de l'individu.

Cette articulation comprend une tête fémorale 14 apte à coopérer avec le cotyle 16 du bassin 18, symbolisé en trait discontinu.

La tête fémorale 14 est portée par l'extrémité 20 proximale du col 22 du fémur tandis que l'extrémité 24 distale du col 22 du fémur est solidaire de l'os 28 long du fémur, la diaphyse. L'axe longitudinal du col 22 du fémur forme un angle α avec l'axe XX' longitudinal de l'os 28 long du fémur.

La tête coxofémorale comprend en outre les trochanters, petit trochanter 30 et grand trochanter 32, définissant un région trochantérienne 34.

Ces différentes régions sont le siège des liaisons avec les muscles.

Cette articulation coxofémorale est soumise à des contraintes mécaniques sévères.

Il existe de multiples possibilités d'impacts traumatiques.

Néanmoins, on peut considérer que les deux raisons principales proviennent d'efforts non supportés, soit orientés suivant la flèche F1, sensiblement verticale, résultant d'un choc, d'une surcharge. L'axe vertical étant considéré avec la personne debout suivant une direction sensiblement parallèle à l'axe longitudinal XX' du fémur.

Cet effort suivant F1 est transmis directement sur la tête fémorale, de façon sensiblement parallèle à l'axe longitudinal XX' de l'os long du fémur.

On note dès lors la création d'un couple de forces.

Or la matière osseuse comme tout matériau présente un limite élastique et une limite plastique avec une force de rupture établie, ceci en fonction de l'état de cette matière osseuse.

Or, lorsque la limite élastique est dépassée, il se produit des lésions, notamment des fissures. Mais, lorsque la résistance à la rupture est atteinte, l'os se brise et en l'occurrence, dans le cas de traumatismes suivant F1 on trouve les fractures du type sous capitale ou basi-cervicale.

Dans le cas d'une chute ou d'un choc accidentel, l'effort est exercé plus latéralement, suivant F2, c'est-à-dire sensiblement dans la région trochantérienne conduisant à des fractures de type pertrochantériennes ou intertrochantériennes.

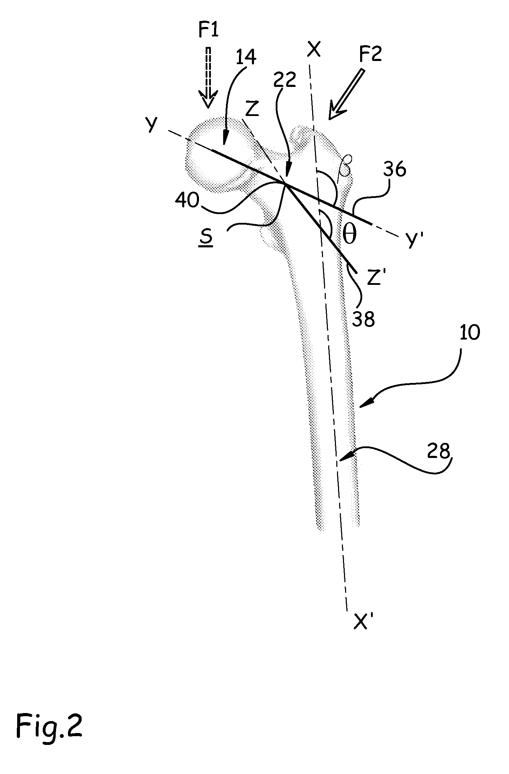

Afin de prévenir les traumatismes de ce type ou éventuellement de les réparer, l'architecture générale du dispositif selon la présente invention est représentée sur la figure 2.

Ce dispositif implantable comprend un premier implant 36 et un second implant 38. Ces implants sont de forme générale tubulaire, creux ou plein, à profil de révolution ou non, avec des dimensions telles que la longueur est très supérieure au diamètre.

Ces deux implants étant sécants en un point S, avec une liaison 40 fixe entre les deux implants, au droit de ce point S d'intersection.

Ces deux implants 36, 38 sont inclinés et voient chacun leur axe longitudinal YY' et ZZ' incliné par rapport à l'axe longitudinal XX' de l'os long du fémur, c'est-à-dire qu'aucun des deux axes YY' et ZZ' n'est confondu avec cet axe longitudinal XX'.

Le premier implant 36 a son axe YY' longitudinal qui fait un angle β avec l'axe XX', sensiblement égal à α, et le second implant a son axe ZZ' longitudinal qui fait un angle θ avec ce même axe XX'. Dans l'architecture représentée, θ est d'une valeur angulaire supérieure à celle de β.

Les angles sont considérés avec la tête coxofémorale en partie haute, sur le fémur gauche, dans le sens horaire, 0° étant en partie haute de l'axe XX', côté X et 180° côté X'.

Les angles θ et β sont compris entre 91 et 179°.

Pour le fémur droit de la même personne, les angles θ et β sont compris entre 181 et 269°.

Plus particulièrement, l'axe YY*'* est sensiblement confondu avec l'axe longitudinal du col du fémur.

Ce premier implant 36 a donc un rôle de raidisseur liant la tête 14 fémorale, le col 22 du fémur et l'os long 28 du fémur. Il augmente la résistance à la flexion.

Ce premier implant 36 doit être positionné afin que son extrémité Y distale vienne dans la tête fémorale sans faire saillie, en deçà de la partie corticale afin de ne pas entraver les mouvements de la surface de la tête fémorale dans le cotyle qui la reçoit. Cette épaisseur corticale est même garante d'une butée éventuelle à la perforation par l'implant en cas de choc important.

L'extrémité Y' proximale de ce premier implant passe à travers la corticale et peut rester en saillie légère, de façon à avoir la certitude d'une bonne reprise des efforts.

En effet, lorsque l'effort est exercé suivant la flèche F1, le premier implant 36 travaille à la flexion, tel un raidisseur, l'extrémité Y' proximale pouvant être considérée comme un encastrement, limitée par la seule résistance mécanique de la corticale qui, dans ce cas, est très élevée puisque la résultante de la réaction s'exerce dans le plan de l'os.

Lorsque l'effort est exercé suivant la flèche F2, le premier implant travaille aussi à la flexion cette fois-ci dans sa partie médiane c'est-à-dire en flexion sur deux appuis, l'un étant la zone distale de l'implant dans la tête fémorale et l'autre appui étant la zone proximale de l'implant dans la corticale.

Le résistance aux efforts suivant F2 dans la zone trochantérienne est également fortement renforcée.

L'invention propose d'augmenter de façon encore beaucoup plus significative cette résistance mécanique en réalisant l'architecture décrite ci-avant avec le second implant 38 qui assure la fonction de jambage.

Ce second implant 38 est donc positionné par sa partie Z' proximale également dans la corticale de l'os long du fémur, en un point situé à une distance plus éloignée de l'articulation coxofémorale.

L'extrémité Z distale du second implant est positionnée au droit du point S d'intersection et forme la liaison 40 fixe. L'extrémité peut se prolonger au-delà si nécessaire.

Le point S d'intersection est situé environ entre la partie médiane du premier implant et l'extrémité Y' distale

On obtient sensiblement une configuration en forme de Y.

Dès lors, on note le rôle de jambage du premier implant 36 par le second implant 38.

Ainsi, lorsque l'effort est exercé suivant F1, la résistance à la flexion du premier implant est renforcée puisque la partie "flexible" reçoit un jambage sensiblement en partie médiane, ce qui raccourcit le bras de levier de la partie comprise entre la partie médiane et la partie proximale.

De plus, on peut considérer qu'il se produit aussi un couple de forces autour du point fixe S. Une majorité de l'effort F1, exercé suivant la direction de la flèche F1, est reprise par la corticale car la force équilibrante du couple s'exerce dans le plan de la corticale de l'os 26 long du fémur dans une direction opposée à F1, allant vers l'articulation coxofémorale.

Quant aux efforts et/ou chocs exercés suivant F2, ils s'exercent ainsi qu'indiqué ci-avant dans la partie médiane justement au droit de point S d'intersection, ce qui reporte les efforts exercés sur le premier implant 36 en partie sur le second implant 38. Ces efforts se diffusent donc jusqu'à la partie périostique de l'os long du fémur, dont la résistance mécanique est la plus importante.

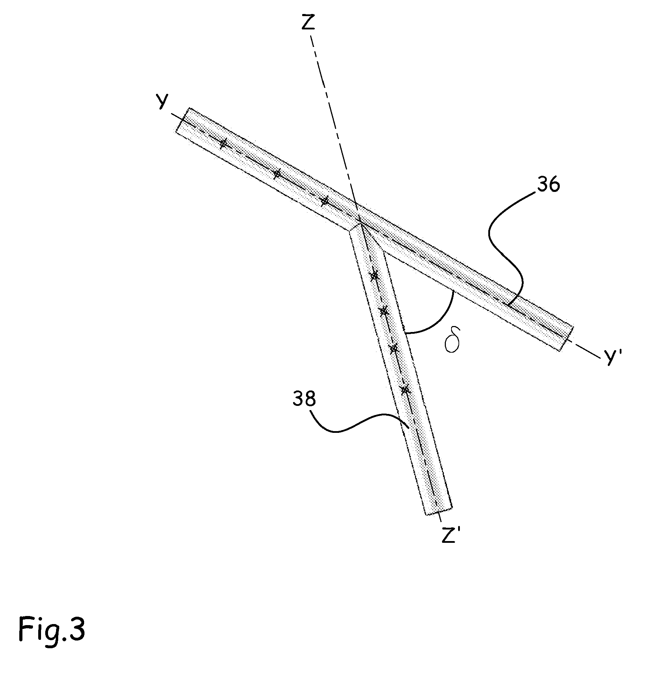

Sur la figure 3, on a représenté schématiquement la liaison 40 fixe pour montrer que l'angle δ d'intersection doit rester constant. Cette liaison mécanique peut prendre différentes formes suivant différents modes de réalisation mais il convient de la considérer comme un encastrement du point de vue résistance des matériaux.

Il n'y a donc pas de mouvement relatif de translation, ni de mouvement relatif angulaire entre les premier 36 et second 38 implants.

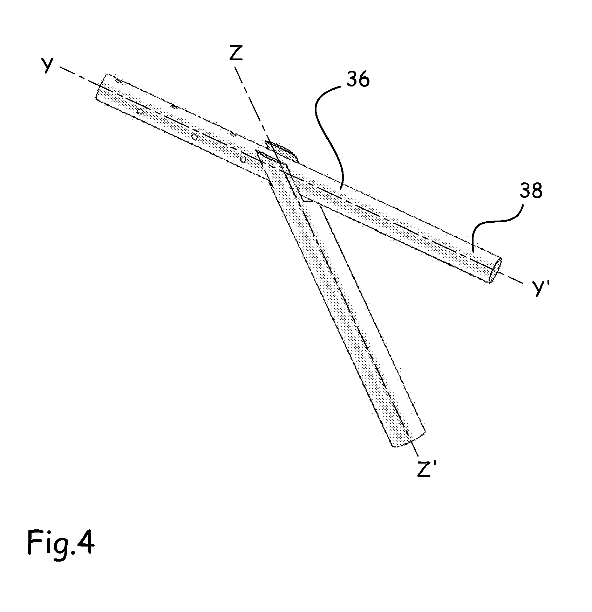

La liaison 40 fixe est représentée sur la figure 4 comme étant une pénétration du premier implant 36 dans le second implant 38 . En l'occurrence le montage prévu est un encliquetage élastique avec des ergots. Il aurait pu s'agir d'une lumière ménagée dans le second implant 38 à travers laquelle passe le premier implant 36 sans que cela ne change la réalisation. Il s'agit de montages totalement équivalents.

Le choix reste à la portée de l'homme de l'art en fonction des besoins, de l'ergonomie de pose ou des contraintes morphologiques du patient.

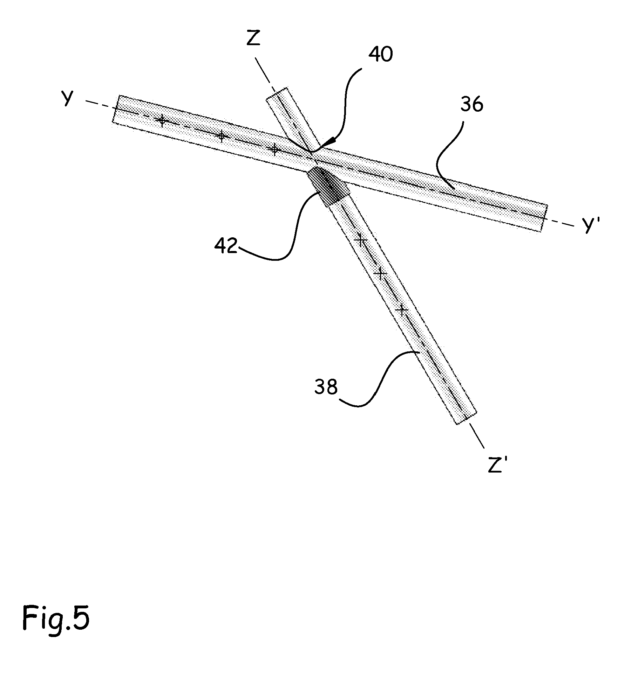

La liaison 40 fixe représentée sur la figure 5 consiste en une pénétration du second implant 38 dans le premier implant 36. Dans un tel agencement, une butée 42, ménagée sur le second implant 38, assure le blocage et la stabilité de la liaison pour obtenir la fonction de jambage. L'angle est donné par le perçage oblique.

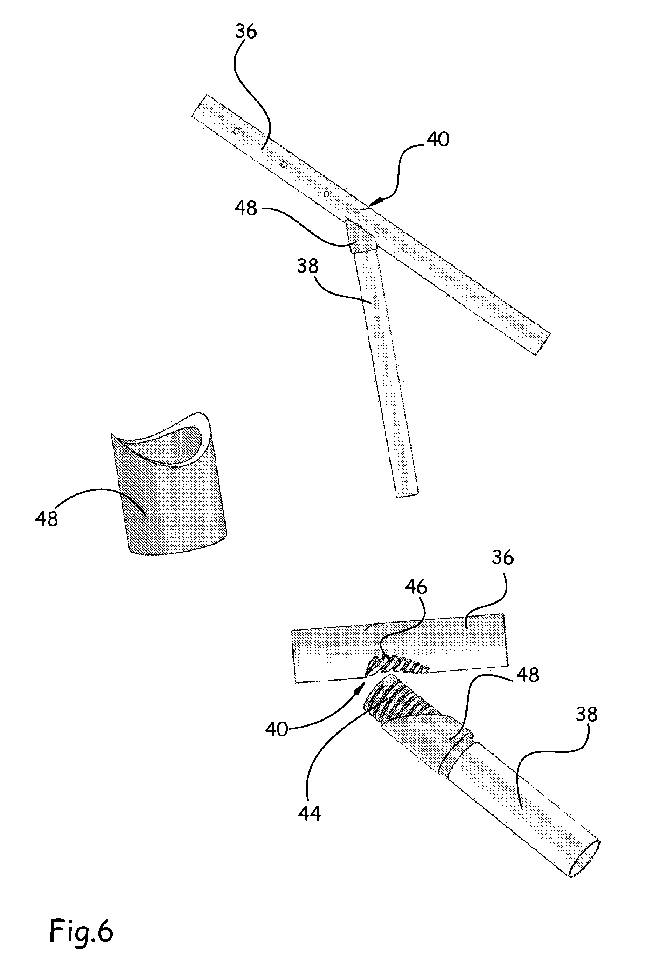

La liaison 40 fixe selon un troisième mode général de réalisation illustré sur les figures 6A, 6B et 6C, consiste à prévoir une liaison à verrouillage mécanique. En l'occurrence, le mode de réalisation représenté consiste en une liaison comprenant un filetage 44 porté par le second implant 38 et un trou 46 taraudé ménagé dans le corps du premier implant 36. Une bague 48 permet de compenser l'écart angulaire et de pourvoir à un appui stable.

La bague est montée libre en rotation et s'oriente pour se positionner par rapport à l'autre implant.

Ces modes de réalisation peuvent prendre de nombreuses autres formes sans sortir du cadre de l' invention, l' homme de l'art pouvant modifier la liaison par vissage au moyen d'un encliquetage, d'un emboîtement conique, d'un emboîtement à coupelles.

Les implants 36 et 38, quel que soit le mode de réalisation, peuvent prendre toute forme adaptée mais de façon générale, ces implants sont réalisés à partir de tubes pleins, creux ou partiellement creux. La section est de dimensions très inférieures à la longueur.

Pour donner un ordre de grandeur, le diamètre de la section est compris entre 3 et 15 mm avec une longueur de 50 à 150 mm.

La section de ces tubes peut aussi prendre toute forme requise par les calculs ou les besoins chirurgicaux : circulaire, elliptique, carrée, hexagonale ou en étoile, filetée extérieurement tout du long ou partiellement ou encore une combinaison de celles-ci. L'implant peut être conique sur toute sa longueur.

En fonction du mode de réalisation retenu, le profil adapté est choisi. S'il est prévu un vissage, la section circulaire de l'implant à visser s'impose, tandis que la section multipans pour le premier implant peut être retenue.

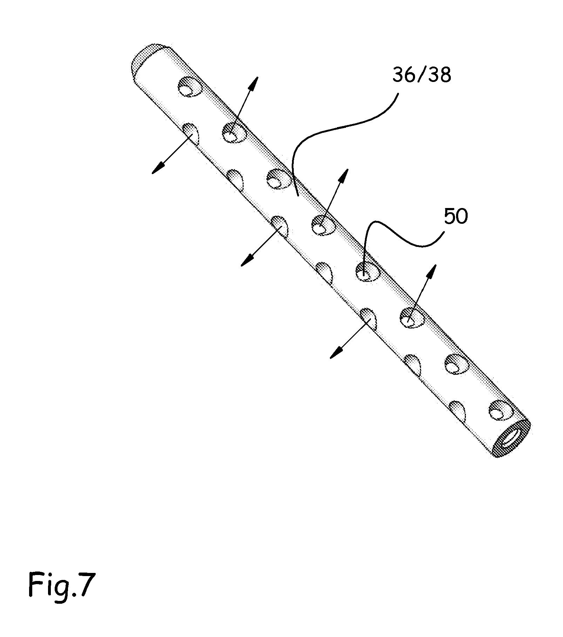

L'extérieur de ces implants peut être lisse, cannelé, avec un profil crénelé ou encore muni d'un filetage, ceci sur l'intégralité de la surface ou partiellement, voire l'extérieur peut présenter une combinaison de ces états de surface. Chaque tube constituant un implant peut aussi présenter des orifices 50 débouchant sur l'extérieur dans le cas des implants totalement ou partiellement creux, comme montré sur la figure 7. Ces orifices permettent une injection de compositions injectables du type ciments biocompatibles, à partir de l'extrémité proximale accessible, une fois l'implant en place. Ces compositions durcissent in situ.

Ces compositions ont un triple but :
- consolidation de la matière spongieuse autour de la zone implantée au moyen de matière injectée qui se diffuse en périphérie de l'orifice,
- immobilisation de l'implant dans la matière osseuse par une surface de contact augmentée, et
- verrouillage de la liaison 40 fixe des deux implants au droit du point S d'intersection.

L'extrémité distale Y et Z est avantageusement chanfreinée ou arrondie pour faciliter la progression à l'introduction.

Le matériaux utilisés sont des matériaux bien connus en chirurgie comme l'acier inoxydable, le titane, les polymères chargés comme la polyéther éther cétone à forte résistance mécanique ou non chargés de façon à se rapprocher de la résistance de l'os.

De même, la surface des implants peut aussi porter un revêtement ou subir un traitement de surface pour améliorer la biocompatibilité, l'intégration dans l'os et le développement des cellules tissulaires, comme l'hydroxyapatite.

Suivant un perfectionnement de l' invention, les implants peuvent être à longueur variable de façon à ajuster leur longueur in situ.

Ainsi, lors de l'implantation, au cours de l'intervention chirurgicale, si le praticien rencontre des difficultés d'insertion par exemple, il lui est possible d'ajuster la longueur de l'implant afin de permettre une adaptation de la longueur en saillie de la partie distale hors de la corticale.

Le montage télescopique peut consister en un implant en deux parties reliées entre elles par une zone à pas de vis.

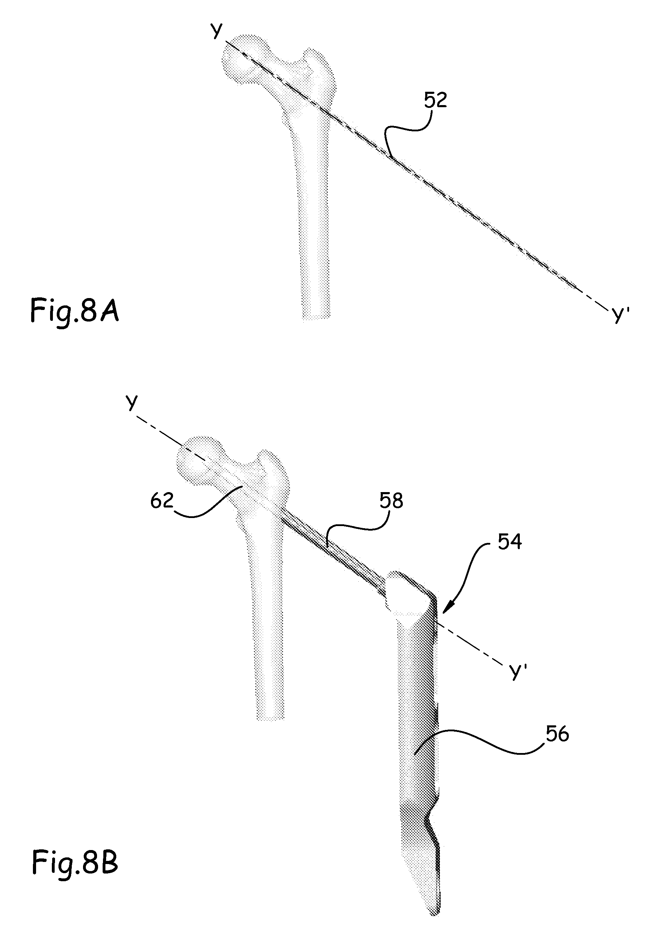

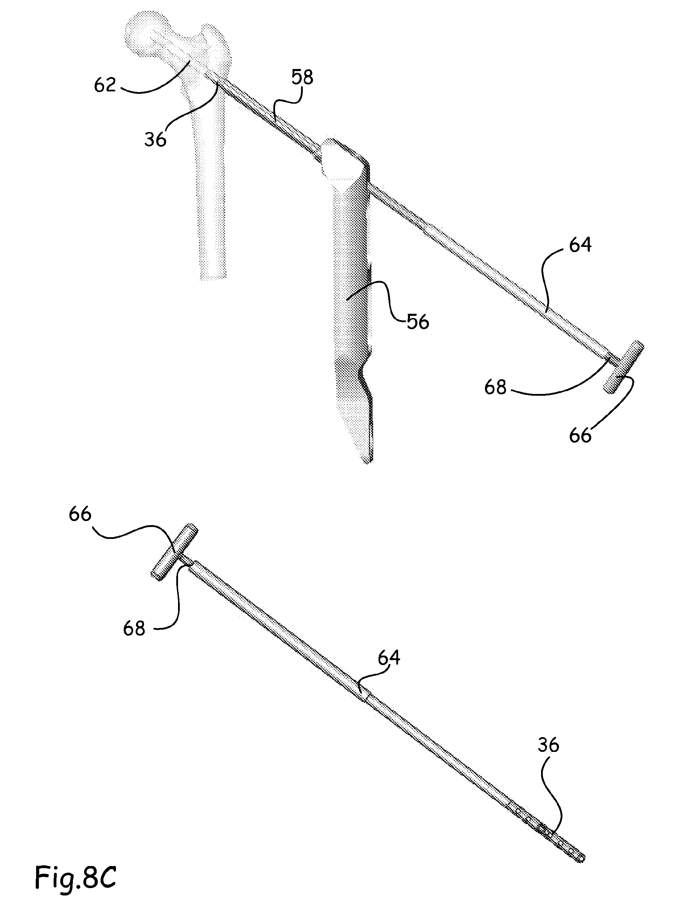

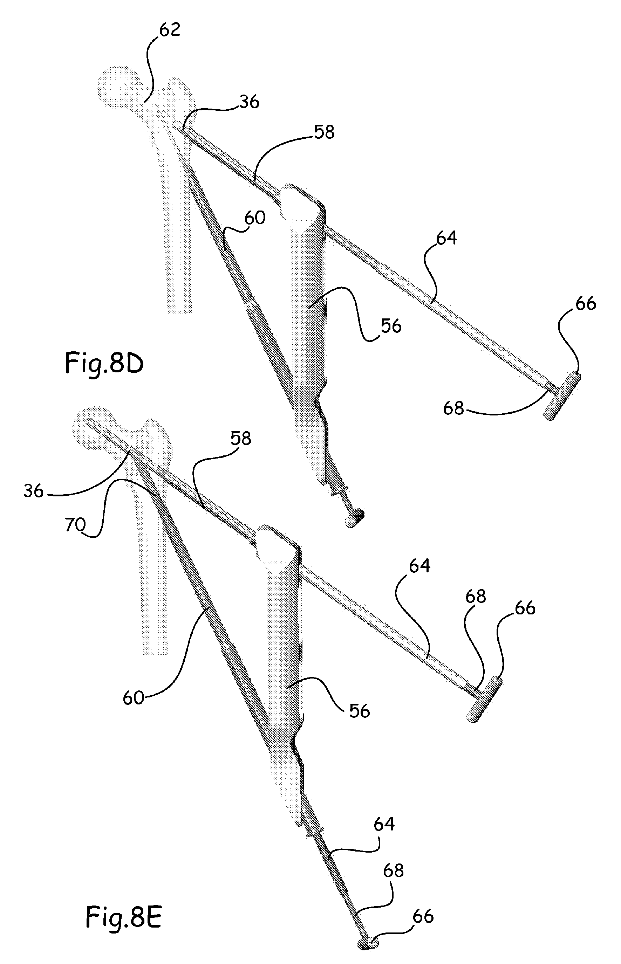

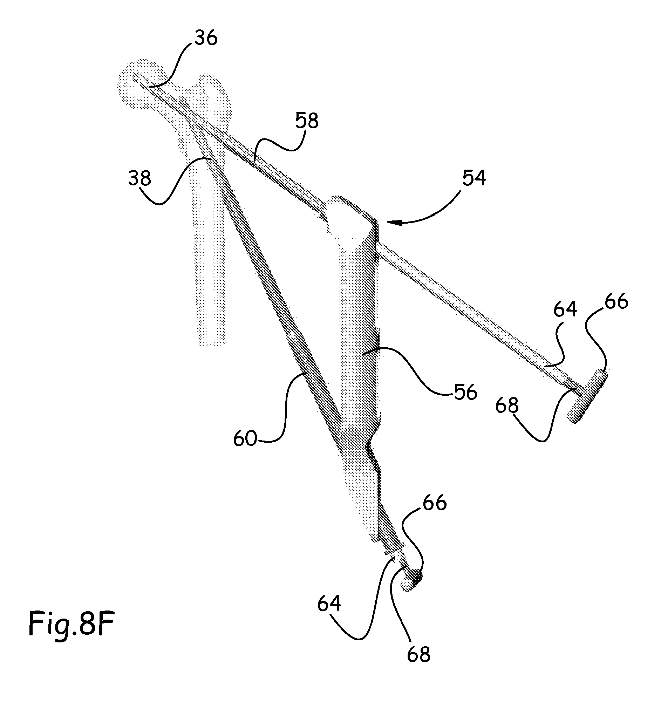

La mise en place du dispositif selon la présente invention est illustrée en accord avec les figures 8.

Cette description devra être adaptée en fonction du mode de réalisation retenu mais les étapes seront sensiblement identiques. De plus les étapes essentielles font l'objet du synoptique sans mentionner toutes les interventions moins essentielles, nécessaires et connues de tout praticien apte à réaliser ce type d'intervention.

Afin de permettre l'intervention du praticien dans des conditions satisfaisantes de sécurité, de sûreté, de qualité et d'ergonomie, il est prévu un ancillaire spécifique qui sera décrit au fur et à mesure du déroulement du synoptique.

La première étape illustrée sur la figure 8A montre l'introduction d'une broche 52, au plus proche de l'axe central du col du fémur. L'introduction est réalisée sous contrôle fluoroscopique par exemple, après incision des tissus mous et du périoste de l'os long du fémur.

L'implantation première de cette broche est importante et doit être vérifiée tant dans le plan frontal que sagittal.

Ensuite, figure 8B, il est fait appel à un sextant 54 pour assurer le positionnement des différents abords et des implants 36 et 38 au terme de l'intervention.

Ce sextant 54 comporte un corps 56 avec une ligne de visée matérialisée par un premier tube 58 de guidage, suivant l'axe de la broche 52 et donc de l'axe YY' du premier implant à mettre en place.

Ce sextant comprend en outre une seconde ligne de visée matérialisée par un second tube 60 de guidage, suivant l'axe ZZ' du second implant à mettre en place, comme cela sera visible sur les figures 8D, 8E et 8F.

Un perçage est effectué suivant l'axe YY' à l'aide d'un foret afin de recevoir ensuite le premier implant 36 dans le logement 62 ainsi réalisé. Le foret de perçage est guidé par le tube 58 de guidage.

La profondeur du perçage est contrôlée par la longueur d'insertion du foret, toujours par rapport au tube 58 de guidage.

La longueur du logement 62 ainsi réalisé étant connue, le choix de la longueur et/ou le réglage de la longueur du premier implant 36 est effectué par le praticien.

Sur la figure 8C, on procède à l'insertion partielle du premier implant 36, suivant un premier protocole.

L'implant 36 est monté sur un préhenseur 64 qui est un tube du diamètre de l'implant. Dans le mode de réalisation retenu, le préhenseur 64 comporte des moyens de fixation amovible, en rotation de l'implant 36. Ces moyens comprennent en l'occurrence une extrémité filetée ménagée sur l'extrémité de l'implant 36 tandis que le préhenseur 64 est muni, à son extrémité proximale, d'un taraudage de profil conjugué. L'implant 36 est ainsi fixé de manière amovible sur le préhenseur 64 mais permettant ainsi au praticien d'agir en translation. Ce préhenseur 64 est prévu pour coopérer avec le sextant 56 et le premier tube 58 de guidage afin d'assurer une continuité d'alignement.

Il est en outre prévu un orienteur 68 qui est une tige de petit diamètre, montée de façon coaxiale interne au préhenseur 64, l'orienteur 68 étant libre en rotation et en translation dans le préhenseur 64.

L'extrémité distale de l'orienteur 68 est muni d'une dent prévue pour s'insérer dans une encoche de profil conjugué ménagée dans la partie proximale de l'implant, permettant ainsi au praticien d'agir en rotation lorsque l'orienteur est totalement introduit. Tout autre moyen d'entraînement en rotation déconnectable entre le préhenseur et l'orienteur peut être envisagé. L'orienteur possède une poignée 66 pour faciliter la manoeuvre par le praticien. Cette opération d'insertion partielle du premier implant 36 étant réalisée suivant le protocole retenu, le second implant 38 peut alors être mis en place à son tour, suivant une autre étape du protocole.

Sur la figure 8D, le praticien procède donc à l'incision des tissus mous et du périoste puis à l'introduction d'une broche, comme précédemment, broche qui est introduite jusqu'à venir déboucher dans le logement 62 au point d'intersection S, et au-delà si nécessaire, ceci grâce à la géométrie du sextant. Le second tube 60 de guidage du sextant permet de suivre le bon angle d'orientation et l'alignement.

Figure 8E, un perçage est effectué à l'aide d'un foret de façon à générer un second logement 70 destiné à recevoir le second implant 38.

Une fois le second logement 70 réalisé, le premier implant 36 est introduit intégralement dans sa position définitive également. Si nécessaire, il est possible d'impacter l'orienteur 68 et de le manipuler en rotation pour parfaire la mise en place et l'insertion complète du premier implant 36 dans son logement 62.

Une fois le premier implant positionné, le second implant 38 est introduit à son tour, voir figure 8F.

Si la liaison 40 fixe au point S d'intersection est du type à vissage par exemple, ainsi qu' illustré sur les figures 6A à 6C, alors au moyen de l'orienteur 68 et de sa poignée 66, le second implant 38 est mis en rotation jusqu'à ce que la liaison 40 fixe, vissée, soit bloquée par vissage complet.

Dès lors les deux implants sont solidarisés et positionnés dans le fémur.

Le matériel ancillaire peut alors être retiré.

Selon un autre protocole, le premier implant peut-être introduit dès sa mise en place, en intégralité, en position définitive.

Ensuite, le perçage du second logement 70 est effectué sans arriver débouchant dans le premier logement 62 puisqu'il est occupé par le premier implant 36. Ensuite le second implant 38 est introduit en totalité et l'introduction de ce second implant pousse les tissus mous sur la partie non forée du logement jusqu'à la connexion avec le premier implant.

Ceci évite une implantation du premier implant en deux étapes et peut être préféré car le premier implant est positionné lorsque les étapes d'implantation du second implant sont conduites.

Si nécessaire, quel que soit le protocole d'implantation suivi, le praticien peut procéder, comme indiqué dans la description qui précède, à une injection d'un matériau biocompatible de scellement et/ou de renforcement et/ou de traitement, moyennant l'introduction dans ladite composition injectée de principes actifs.

La description qui vient d'être présentée vise particulièrement une application au fémur mais pourrait aussi trouver une application dans le cas de fractures de la malléole fibulaire ou tibiale, de fractures du pilon tibial, de fractures de la main ou du pied.

## Revendications

1. Dispositif implantable pour le traitement préventif ou curatif de l'articulation (12) coxofémorale comprenant le fémur (10) avec son os long (28) et sa paroi corticale, définissant un axe longitudinal XX', une tête fémorale (14), un col (22) du fémur et un axe longitudinal sécant avec l'axe longitudinal XX' formant un angle α, comprenant:
- Un premier implant (36) dont l'axe YY' est prévu pour être agencé sensiblement suivant ledit axe longitudinal dudit col (22), avec une extrémité Y distale non débouchante et une extrémité Y' proximale débouchante à travers la paroi corticale,
- Un second implant (38) dont l'axe ZZ' est prévu pour être sécant avec l'axe YY' dudit premier implant (36) en un point d'intersection S à proximité de son extrémité distale Z et prévu pour être sécant avec l'axe longitudinal XX' du fémur, l'extrémité Z' proximale traversant la paroi corticale dudit fémur, et
- Une liaison (40) fixe des implants (36) et (38) au point S' **caractérisé en ce que** lextrémité Z' proximale du second implant est située à une distance plus éloignée de l'articulation coxofémorale que l'extrémité Y' proximale débouchante du premier implant.

2. Dispositif implantable pour le traitement préventif ou curatif de fractures du fémur selon la revendication 1, **caractérisé en ce que** le premier implant (36) a son axe YY' longitudinal qui fait un cingle β avec l'axe XX' et le second implant (38) a son axe ZZ' longitudinal qui fait un angle θ avec ce même axe XX', θ étant d'une valeur angulaire supérieure à celle de β.

3. Dispositif implantable pour le traitement préventif ou curatif de fractures du fémur selon la revendication 1 ou 2, **caractérisé en ce que** les extrémités proximales de chaque implant (36,38) sont en saillie par rapport à la corticale de l'os long (18) du fémur (10).

4. Dispositif implantable pour le traitement préventif ou curatif de fractures du fémur selon l'une quelconque des revendications précédentes, **caractérisé en ce que** les angles β et θ sont compris entre 91 et 179° pour le fémur gauche et entre 181 et 269° pour le fémur droit.

5. Dispositif implantable pour le traitement préventif ou curatif de fractures du fémur selon l'une quelconque des revendications précédentes, **caractérisé en ce que** la liaison (40) fixe au point d'intersection S est une pénétration du premier implant (36) dans le second implant (38).

6. Dispositif implantable pour le traitement préventif ou curatif de fractures du fémur selon l'une quelconque des revendications 1 à 4, **caractérisé en ce que** la liaison (40) fixe au point d'intersection S est une pénétration du second implant (38) dans le premier implant (36).

7. Dispositif implantable pour le traitement préventif ou curatif de fractures du fémur selon la revendication 6, **caractérisé en ce que** la liaison (40) fixe au point d'intersection S est une liaison à verrouillage mécanique comprenant un filetage (44) porté par le second implant (38) et un trou (46) taraudé ménagé dans le corps du premier implant (36) ainsi qu'une bague (48) de compensation de l'écart angulaire et d'appui stable.

8. Dispositif implantable pour le traitement préventif ou curatif de fractures du fémur selon l'une quelconque des revendications précédentes, **caractérisé en ce que** les implants (36,38) sont des tubes pleins, creux ou partiellement creux dont la section est de dimensions très inférieures à la longueur.

9. Dispositif implantable pour le traitement préventif ou curatif de fractures du fémur selon l'une quelconque des revendications précédentes, **caractérisé en ce que** chaque implant (36,38), réalisé à partir d'un tube au moins partiellement creux, comprend au moins un orifice (50) d'injection d'une composition injectable du type ciment biocompatible, à partir de l'extrémité proximale accessible, une fois ledit implant (36,38) en place.

10. Dispositif implantable pour le traitement préventif ou curatif de fractures du fémur selon l'une quelconque des revendications 8 ou 9, **caractérisé en ce que** la section du tube constituant chaque implant est circulaire, elliptique, carrée, hexagonale, en étoile, conique sur toute la longueur ou partiellement, fileté sur toute sa longueur ou partiellement ou encore une combinaison de celles-ci.

11. Dispositif implantable pour le traitement préventif ou curatif de fractures du fémur selon l'une quelconque des revendications précédentes, **caractérisé en ce que** l'un au moins des implants (36,38) est à longueur variable de façon à ajuster sa longueur un situ.

12. Dispositif implantable pour le traitement préventif ou curatif de fractures du fémur selon l'une quelconque des revendications précédentes, **caractérisé en ce que** la surface des implants (36,38) porte un revêtement ou subit un traitement de surface pour améliorer la biocompatibilité et le développement des cellules tissulaires.

13. Ancillaire pour la mise en place du dispositif implantable pour le traitement préventif ou curatif de fractures du fémur selon l'une quelconque des revendications précédentes, **caractérisé en ce qu'**il comprend :
- un sextant (54) pour assurer le positionnement des différents abords et des implants (36, 38), comprenant un corps (56) avec
∘ une première ligne de visée matérialisée par un premier tube (58) de guidage, suivant l'axe YY' du premier implant (36) à mettre en place, et
∘ une seconde ligne de visée matérialisée par un second tube (60) de guidage, suivant l'axe ZZ' du second implant (38) à mettre en place
- un préhenseur (64) sous forme d'un tube, qui comporte des moyens de fixation amovible, en translation et en rotation, d'un implant (36,38),
- un orienteur (68), sous forme d'une tige, montée de façon coaxiale interne dans ledit préhenseur (64), l'orienteur étant libre en rotation et en translation dans le préhenseur (64), cet orienteur (68) comportant une poignée (66) de manoeuvre, et
- des moyens d'entraînement en rotation déconnectables entre le préhenseur (64) et l'orienteur (68).

## Patentansprüche

1. Implantierbare Vorrichtung für die präventive oder kurative Behandlung des Hüftgelenks (12), das den Oberschenkelknochen (10) mit seinem langen Schaft (28) und seiner kortikalen Wand, die eine Längsachse XX' bilden, einen Oberschenkelkopf (14), einen Hals (22) des Oberschenkelknochens und eine sich mit der Längsachse XX' unter dem Winkel α schneidende Längsachse umfasst, mit:
- einem ersten Implantat (36), dessen Achse YY' dazu vorgesehen ist, im Wesentlichen längs der Längsachse des Halses (22) ausgerichtet zu sein, mit einem nicht hervortretenden distalen Ende Y und einem durch die kortikale Wand hervortretenden proximalen Ende Y',
- einem zweiten Implantat (38), dessen Achse ZZ' dazu vorgesehen ist, sich in der Nähe seines distalen Endes Z mit der Achse YY' des ersten Implantats (36) in einem Schnittpunkt S zu schneiden, und dazu vorgesehen ist, sich mit der Längsachse XX' des Oberschenkelknochens zu schneiden, wobei das proximale Ende Z' die kortikale Wand des Oberschenkelknochens durchquert und
- eine feste Verbindung (40) der Implantate (36) und (38) im Punkt S, **dadurch gekennzeichnet, dass** das proximale Ende Z' des zweiten Implantats sich in einer größeren Entfernung vom Hüftgelenk befindet als das hervortretende proximale Ende Y' des ersten Implantats.

2. Implantierbare Vorrichtung für die präventive oder kurative Behandlung von Brüchen des Oberschenkelknochens nach Anspruch 1, **dadurch gekennzeichnet, dass** das erste Implantat (36) die Längsachse YY' hat, die einen Winkel β mit der Achse XX' bildet, und dass das zweite Implantat (38) die Längsachse ZZ' hat, die einen Winkel θ mit der gleichen Achse XX' bildet, wobei θ einen größeren Winkelwert hat als β.

3. Implantierbare Vorrichtung für die präventive oder kurative Behandlung von Brüchen des Oberschenkelknochens nach Anspruch 1 oder 2, **dadurch gekennzeichnet, dass** die proximalen Enden jedes Implantats (36, 38) bezüglich der Kortikalis des langen Schafts (18) des Oberschenkelknochens (10) vorstehen.

4. Implantierbare Vorrichtung für die präventive oder kurative Behandlung von Brüchen des Oberschenkelknochens nach einem der vorhergehenden Ansprüche, **dadurch gekennzeichnet, dass** die Winkel β und θ zwischen 91° und 179° für den linken Oberschenkelknochen und zwischen 181° und 269° für den rechten Oberschenkelknochen liegen.

5. Implantierbare Vorrichtung für die präventive oder kurative Behandlung von Brüchen des Oberschenkelknochens nach einem der vorhergehenden Ansprüche, **dadurch gekennzeichnet, dass** die feste Verbindung (40) am Schnittpunkt S ein Einfügen des ersten Implantats (36) in das zweite Implantat (38) ist.

6. Implantierbare Vorrichtung für die präventive oder kurative Behandlung von Brüchen des Oberschenkelknochens nach einem der Ansprüche 1 bis 4, **dadurch gekennzeichnet, dass** die feste Verbindung (40) am Schnittpunkt S ein Einfügen des zweiten Implantats (38) im ersten Implantat (36) ist.

7. Implantierbare Vorrichtung für die präventive oder kurative Behandlung von Brüchen des Oberschenkelknochens nach Anspruch 6, **dadurch gekennzeichnet, dass** die feste Verbindung (40) am Schnittpunkt S eine mechanische Verriegelung ist, die ein am zweiten Implantat (38) ausgebildetes Gewinde (44) und eine im Körper des ersten Implantats (36) ausgebildete Gewindebohrung (46) aufweist, ebenso wie eine Hülse (48) für die Kompensation des Winkelabstands und als stabile Stütze.

8. Implantierbare Vorrichtung für die präventive oder kurative Behandlung von Brüchen des Oberschenkelknochens nach einem der vorhergehenden Ansprüche, **dadurch gekennzeichnet, dass** die Implantate (36, 38) Vollrohre, hohle Rohre oder teilweise hohle Rohre sind, deren Querschnittsabmessungen weit unterhalb der Länge liegen.

9. Implantierbare Vorrichtung für die präventive oder kurative Behandlung von Brüchen des Oberschenkelknochens nach einem der vorhergehenden Ansprüche, **dadurch gekennzeichnet, dass** jedes Implantat (36, 38), das ausgehend von einem wenigstens teilweisen hohlen Rohr bewerkstelligt wird, wenigstens eine Injektionsöffnung (50) für eine injizierbare Zusammenstellung von der Art eines biokompatiblen Zements aufweist, die nach dem Einbringen des Implantats (36, 38) vom proximalen Ende zugänglich ist.

10. Implantierbare Vorrichtung für die präventive oder kurative Behandlung von Brüchen des Oberschenkelknochens nach einem der Ansprüche 8 oder 9, **dadurch gekennzeichnet, dass** der Querschnitt des das jeweilige Implantat bildenden Rohres kreisförmig, elliptisch, rechtwinklig, hexagonal, standförmig, konisch über seine gesamte Länge oder einen Teil davon ist, über seine gesamte Länge oder einen Teil davon mit einem Gewinde versehen ist, oder eine Kombination von diesem aufweist.

11. Implantierbare Vorrichtung für die präventive oder kurative Behandlung von Brüchen des Oberschenkelknochens nach einem der vorhergehenden Ansprüche, **dadurch gekennzeichnet, dass** wenigstens eines der Implantate (36, 38) von einer variablen Länge ist, um seine Länge vor Ort einzustellen.

12. Implantierbare Vorrichtung für die präventive oder kurative Behandlung von Brüchen des Oberschenkelknochens nach einem der vorhergehenden Ansprüche, **dadurch gekennzeichnet, dass** die Oberfläche der Implantate (36, 38) eine Beschichtung trägt oder einer Oberflächenbehandlung unterzogen ist, um die Biokompatibilität und die Entwicklung von Gewebezellen zu verbessern.

13. Chirurgisches Instrument für die Platzierung der implantierbaren Vorrichtung für die präventive oder kurative Behandlung von Brüchen des Oberschenkelknochens nach einem der vorhergehenden Ansprüche, **dadurch gekennzeichnet, dass** dieses umfasst:
- einen Sextanten (54), um die Positionierung der verschiedenen Hilfsmittel und der Implantate (36, 38) zu gewährleisten, der einen Körper (56) aufweist, mit
∘ einer ersten Visierlinie, die von einer ersten Führungsröhre (58) bereitgestellt ist, die der Achse YY' des zu platzierenden ersten Implantats (36) folgt, und
∘ einer zweiten Visierlinie, die durch eine zweite Führungsröhre (60) bewerkstelligt ist, die der Achse ZZ' des zu platzierenden zweiten Implantats (38) folgt
- einen Greifer (64) in der Gestalt eines Rohres, das in Translationsrichtung und Rotationsrichtung entfernbare Haltemittel eines Implantats (36, 38) umfasst,
- einen Ausrichter (68) in Gestalt einer Stange, die koaxial im Greifer (64) angebracht ist, wobei der Ausrichter im Greifer (64) frei drehbar und schiebbar ist, und der Ausrichter (68) einen Betätigungsgriff (66) umfasst, und
- trennbare Drehantriebsmittel zwischen dem Greifer (64) und dem Ausrichter (68).

## Claims

1. An implantable device for the preventive or curative treatment of the coxofemoral joint (12) comprising the femur (10) with its long bone (28) and its cortical wall, defining a longitudinal axis XX', a femoral head (14), a neck (22) of the femur and a longitudinal axis secant with the longitudinal axis XX' forming an angle α, comprising:
- a first implant (36), the axis YY' of which is designed to be arranged substantially along said longitudinal axis of said neck (22), with a non-emerging distal end Y and a proximal end Y' emerging through the cortical wall,
- a second implant (38), the axis ZZ' of which is designed to be secant with the axis YY' of said first implant (36) at a point of intersection S close to its distal end Z and designed to be secant with the longitudinal axis XX' of the femur, the proximal end Z' passing through the cortical wall of said femur, and
- a fixed connection (40) of the implants (36) and (38) at point S, **characterised in that** the proximal end Z' of the second implant is situated at a distance further from the coxofemoral joint than the emerging proximal end Y' of the first implant.

2. An implantable device for the preventive or curative treatment of fractures of the femur according to claim 1, **characterised in that** the first implant (36) has its longitudinal axis YY' forming an angle β with the axis XX' and the second implant (38) has its longitudinal axis ZZ' forming an angle θ with this same axis XX', θ having an angular value greater than that of β.

3. An implantable device for the preventive or curative treatment of fractures of the femur according to claim 1 or 2, **characterised in that** the proximal ends of each implant (36, 38) project with respect to the cortex of the long bone (18) of the femur (10).

4. An implantable device for the preventive or curative treatment of fractures of the femur according to any one of the preceding claims, **characterised in that** the angles β and θ are between 91° and 179° for the left femur and between 181° and 269° for the right femur.

5. An implantable device for the preventive or curative treatment of fractures of the femur according to any one of the preceding claims, **characterised in that** the connection (40) fixed at the point of intersection S is a penetration of the first implant (36) in the second implant (38).

6. An implantable device for the preventive or curative treatment of fractures of the femur according to any one of claims 1 to 4, **characterised in that** the connection (40) fixed at the point of intersection S is a penetration of the second implant (38) in the first implant (36).

7. An implantable device for the preventive or curative treatment of fractures of the femur according to claim 6, **characterised in that** the connection (40) fixed at the point of intersection S is a mechanical-locking connection comprising a thread (44) carried by the second implant (38) and a threaded hole (46) provided in the body of the first implant (36) as well as a ring (48) for compensating for the angular difference and for stable support.

8. An implantable device for the preventive or curative treatment of fractures of the femur according to any one of the preceding claims, **characterised in that** the implants (36, 38) are solid, hollow or partially hollow tubes, the cross-section of which has dimensions very much less than the length.

9. An implantable device for the preventive or curative treatment of fractures of the femur according to any one of the preceding claims, **characterised in that** each implant (36, 38), produced from an at least partially hollow tube, comprises at least one orifice (50) for injecting an injectable composition of the biocompatible cement type, from the accessible proximal end, once said implant (36, 38) is in place.

10. An implantable device for the preventive or curative treatment of fractures of the femur according to either one of claims 8 or 9, **characterised in that** the cross-section of the tube constituting each implant is circular, elliptical, square, hexagonal, star-shaped or conical over the entire length or partially, threaded over its entire length or partially or a combination of these.

11. An implantable device for the preventive or curative treatment of fractures of the femur according to any one of the preceding claims, **characterised in that** at least one of the implants (36, 38) has a variable length so as to enable adjustment of its length in situ.

12. An implantable device for the preventive or curative treatment of fractures of the femur according to any one of the preceding claims, **characterised in that** the surface of the implants (36, 38) carries a coating or undergoes a surface treatment for improving biocompatibility and the development of tissue cells.

13. An accessory for fitting the implantable device for the preventive or curative treatment of fractures of the femur according to any one of the preceding claims, **characterised in that** it comprises:
- a sextant (54) for positioning the various approaches and implants (36, 38), comprising a body (56) with
∘ a first line of sight represented by a first guide tube (58), along the axis YY' of the first implant (36) to be fitted, and
∘ a second line of sight represented by a second guide tube (60), along the axis ZZ' of the second implant (38) to be fitted,
- a gripper (64) in the form of a tube, which comprises means for the removable fixing, with respect to translation and rotation, of an implant (36, 38)
- an orienter (68), in the form of a rod, mounted so as to be internally coaxial in said gripper (64), the orienter being free to rotate and translate in the gripper (64), this orienter (68) comprising a manoeuvring handle (66), and
- disconnectable means for rotation between the gripper (64) and the orienter (68).
